(19) Europäisches Patentamt
European Patent Office
Office européen des brevets

(11) Veröffentlichungsnummer: **0 450 356 A1**

(12) **EUROPÄISCHE PATENTANMELDUNG**

(21) Anmeldenummer: **91103877.6**

(22) Anmeldetag: **14.03.91**

(51) Int. Cl.5: **C07C 227/18**, C07C 229/08, C07D 263/44

(30) Priorität: **06.04.90 DE 4011171**

(43) Veröffentlichungstag der Anmeldung:
**09.10.91 Patentblatt 91/41**

(84) Benannte Vertragsstaaten:
**AT BE CH DE DK ES FR GB GR IT LI NL SE**

(71) Anmelder: **DEGUSSA AG**
**Weissfrauenstrasse 9**
**W-6000 Frankfurt (Main)(DE)**

(72) Erfinder: **Drauz, Karlheinz, Dr.**
**Zur Marienruhe 13**
**W-6463 Freigericht 1(DE)**
Erfinder: **Knaup, Günter, Dr.**
**Friedhofstrasse 8**
**W-6454 Bruchköbel(DE)**

(54) **Herstellung von alpha-Aminosäure-2,2,2,-trifluorethylester.**

(57) α-Aminosäure-2,2,2-trifluorethylester der allgemeinen Formel I

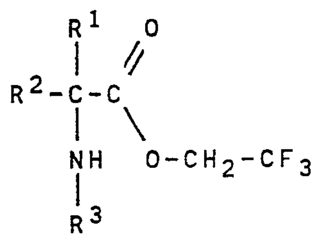

I

sowie ihrer Säureadditionssalze, worin

$R^1$ Wasserstoff oder Alkyl,

$R^2$ Wasserstoff-, Alkyl-, Aryl-, Heteroaryl-, Arylalkyl-, Heteroarylalkyl-, Cycloalkyl- oder Cycloalkylmethyl ist,
oder
$R^1$ und $R^2$ zusammen mit dem sie verbindenden C-Atom einen gesättigten Ring bilden,

$R^3$ Wasserstoff oder Alkyl ist, oder $R^2$ und $R^3$ zusammen mit dem sie verbindenden N- und C-Atom einen gesättigten Ring bilden und für den Fall von $R^1 \neq R^2$ Racemate oder Enantiomere entstehen. Weiterhin wird ein Verfahren zur Herstellung dieser Verbindungen beschrieben.
Verbindungen der Formel I lassen sich als Zwischenprodukte zur Herstellung von Agrochemikalien oder von pharmazeutischen Wirkstoffen verwenden.

EP 0 450 356 A1

Die vorliegende Erfindung betrifft neue α-Aminosäure-2,2,2-trifluorethylester sowie ihre Säureadditionssalze, Verfahren zu ihrer Herstellung und ihre Verwendung.

α-Aminosäure-2,2,2,-trifluorethylester lassen sich nicht durch direkte Veresterung der α-Aminosäuren mit 2,2,2-Trifluorethanol herstellen. Von dieser Verbindungsklasse ist bisher nur das Alanylderivat beschrieben (V.F. Pozdnev, Bioorg. Khim., 10, 912 -20 (1984)), das über eine komplizierte, mehrstufige Synthese hergestellt wurde. Eine einfache Synthese dieser Verbindungsklasse war wünschenswert, da die Verbindungen als wertvolle Zwischenprodukte zur Herstellung von Wirkstoffen z.B. Agrochemikalien dienen können (siehe parallele Anmeldung P 40 11 172.5).

Überraschenderweise wurde nun gefunden, daß α-Aminosäure-2,2,2-trifluorethylester durch Umsetzung der einfach zugänglichen N-Carbonsäurenanhydride mit 2,2,2-Trifluorethanolaten in einer einstufigen Reaktion in guten Ausbeuten hergestellt werden können.

Gegenstand der Erfindung sind deshalb α-Aminosäure-2,2,2-trifluorethylester der allgemeinen Formel I

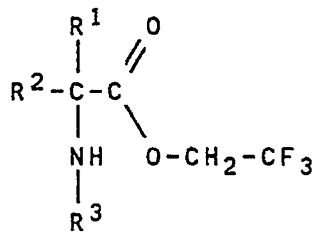

I

worin $R^1$ Wasserstoff oder $C_{1-4}$-Alkyl,

$R^2$ Wasserstoff, Alkyl, substituiertes Alkyl, Aryl, substituiertes Aryl, Heteroaryl, Arylalkyl, Heteroarylalkyl, Cycloalkyl- oder Cycloalkylmethyl ist,

oder

$R^1$ und $R^2$ zusammen mit dem sie verbindenden C-Atom einen 3 bis 7-gliedrigen gesättigten Ring bilden,

$R^3$ Wasserstoff oder $C_{1-4}$-Alkyl ist, mit der Maßgabe, daß für den Fall von $R^1$ = $CH_3$ $R^2$ und $R^3$ nicht Wasserstoff sind

oder

$R^2$ und $R^3$ zusammen mit dem sie verbindenden N- und C-Atom einen 4 bis 7-gliedrigen gesättigten Ring bilden

und

für den Fall $R^1 \neq R^2$ Racemate oder Enantiomere entstehen sowie die Säureadditonssalze davon.

Alkylgruppen können geradkettig oder verzweigt sein, bevorzugt sind Kettenlängen von $C_1$-$C_{12}$ bei geraden Ketten bzw. Kettenlängen von $C_{3-12}$ bei verzweigten Ketten, besonders bevorzugt Kettenlängen von $C_{1-6}$ bei geraden Ketten bzw. $C_{3-6}$ bei verzweigten Ketten, Beispiele sind Methyl, Ethyl, Propyl, Isopropyl, Butyl, Isooctyl, Dodecyl.

Diese Alkylgruppen können gegebenenfalls substituiert sein durch 1-3 Amino- und/oder Hydroxyl- und/oder Halogen-Gruppen, wobei die Aminogruppen durch Acyl- oder Urethan-Gruppen und die Hydroxylgruppen durch $C_{1-4}$-Alkyl- oder Benzyl-Gruppen geschützt sein können. Bevorzugt sind als Acyl-Gruppen Acetyl und Benzoyl, als Urethan-Gruppen Methoxycarbonyl und Benzyloxycarbonyl.

Arylgruppen können bevorzugt Phenyl- oder substituierte Phenyl-Gruppen sein.

Substituierte Arylgruppen sind bevorzugt Mono-, Di-, oder Trihalogen-, Mono- oder Di-trifluormethyl-, Mono-, Di-, Trialkyl-Phenylgruppen, wobei Halogen Fluor Chlor oder Brom ist, Alkyl $C_{1-4}$-Alkyl, bevorzugt Methyl oder Ethyl.

Heteroaryl-Gruppen sind bevorzugt 5 und 6-Ring-Systeme mit 1-2 Heteroatomen im Ring, die O, N, oder S sein können.

Arylalkyl ist bevorzugt Benzyl.

Cycloalkyl und Cycloalkylmethyl sind bevorzugt $C_{3-7}$-Ring-Systeme.

Die Verbindungen der Formel I bilden mit Säuren Salze, die ebenfalls Gegenstand der Erfindung sind. Beispiele solcher Salze sind Salze mit Mineralsäuren wie Salzsäure, Bromwasserstoffsäure, Schwefelsäure, Phosphorsäure oder organischen Säuren wie Oxalsäure, Äpfelsäure, Essigsäure, Propionsäure, Zitronensäure, Sulfonsäuren, Phosphonsäuren.

Bevorzugt sind Salze der Salzsäure.

Ein weiterer Gegenstand der Erfindung ist ein Verfahren zur Herstellung von α-Aminosäure-2,2,2-trifluorethylestern der allgemeinen Formel I, das dadurch gekennzeichnet ist, daß man ein N-Carbonsäureanhydrid der allgemeinen Formel II,

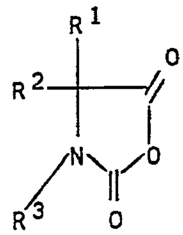

II

in der $R^1$, $R^2$, $R^3$ die unter Anspruch 1 bis 3 angegebene Bedeutung haben, wobei $R^2$ und $R^3$ auch Wasserstoff sein können, wenn $R^1$ = $CH_3$ ist, mit Lithium-, Natrium- oder Kalium-2,2,2-trifluorethanolat umsetzt.

Die Reaktion wird am zweckmäßigsten so durchgeführt, daß aus 2,2,2-Trifluorethanol und einem Alkalimetall, vorzugsweise Natrium, eine Trifluorethanolat-Lösung bereitet wird, zu der man eine Lösung des N-Carbonsäureanhydrides der allgemeinen Formel II in einem organischen Lösungsmittel wie z.B. einem Ether, Carbonsäureester, (halogeniertem) Kohlenwasserstoff, Carbonsäurenitril, Carbonsäureamid bei -20 °C bis +40 °C, vorzugsweise bei -10 °C bis +20 °C , gibt. Die Herstellung der Verbindungen der allgemeinen Formel II erfolgt nach bekannten Literaturverfahren (H.R. Kricheldorf, "α-Aminoacid-N-Carboxy-Anhydrides and Related, Hetereocycles", Springer Verlag, Berlin, Heidelberg, 1987, Seite 3 - 51).

Zur Isolierung der 2,2,2-Trifluorethylester der allgemeinen Formel I erwies es sich am zweckmäßigsten, die Reaktionsmischung nach Beendigung der Reaktion (2 - 30 min im oben genannten Temperaturbereich, vorzugsweise nach 10 min bei 0 °C) mit einer Säure, wie z.B. Salzsäure, anzusäuern und die Säureadditionssalze nach Abdampfen der Lösungsmittel unter vermindertem Druck aus den festen Rückständen durch Digerieren mit einem geeigneten Lösungsmittel wie z.B. Essigsäureethylester, Chloroform, Dichlormethan, herauszulösen. Nach Abfiltrieren der anorganischen Salze und Entfernen des Lösungsmittels fallen die Säureadditionssalze der Verbindungen der allgemeinen Formel I in fester Form an. Die freien Ester sind daraus leicht durch Versetzung der Salze mit einer Base wie z.B. einem tert. Amin und Abtrennung der Ammoniumsalze herstellbar.

Verbindungen der allgemeinen Formel I können als Zwischenprodukte zur Herstellung von Agrochemikalien und von pharmazeutischen Wirkstoffen dienen.

Die Herstellung der Hydrochlorid-Verbindungen nach Anspruch 1 bis 4 wird an den folgenden Beispielen erläutert.

Allgemeine Herstellvorschrift für die Hydrochloride der α-Aminosäuren-2,2,2-trifluorethylester der Formel I:

Aus 85 ml 2,2,2-Trifluorethanol und 0,1 mol Natrium wird eine Natrium-2,2,2-trifluorethanolat-Lösung hergestellt. Zu dieser Lösung gibt man bei 0 °C innerhalb von maximal 5 min eine Lösung von 0,085 mol des N-Carbonsäureanhydrides in 200 ml Tetrahydrofuran. Nach 10 min säuert man mit 190 ml 1 N Salzsäure an und destilliert das Lösungsmittel unter vermindertem Druck ab. Die α-Aminosäure-2,2,2-trifluorethylester Hydrochloride werden durch Digerieren mit Chloroform oder Essigsäureethylester vom Natriumchlorid abgetrennt.

Nach Abdestillieren des Lösungsmittels erhält man die Hydrochloride der 2,2,2-Trifluorethylester als kristalline Verbindungen. Ausbeuten und Daten ausgewählter Verbindungen sind in Tabelle 1 zusammengefaßt.

Tabelle: Erzielte Ausbeuten und Daten einiger α-Aminosäure-2,2,2-trifluorethylester Hydrochloride

| Bsp. | $R^1$ | $R^2$ | $R^3$ | Stereo-chemie | Ausb. | Schmp. | $^1$H-NMR δ [ppm] | Analyse |
|---|---|---|---|---|---|---|---|---|
| 1 | H | $(CH_3)_2CH$ | H | L | 46 % | 174 - 76°C | (DMSO-$d_6$): 0,95 (d, 3H), 1,01 (d, 3H), 2,27 (m, 1H) 4,02 (d, 1H), 4,95 (m, 2H) 8,88 (br.s, 3H) | Ber.: C 35,68 H 5,56 N 5,95<br>Gef.: C 35,71 H 5,70 N 6,02 |
| 2 | H | $(CH_3)_2CHCH_2$ | H | L | 81 % | 131 - 33°C | ($CDCl_3$): 0,98 (d, 3H), 1,10 (d, 3H), 1,53 (m, 2H), 2,28 (m, 1H), 4,25 (d, 1H), 4,49 (m, 1H), 4,78 (m, 1H), 8,50 (br.s, 3H) | Ber.: C 38,49 H 6,06 N 5,61<br>Gef.: C 38,52 H 6,15 N 5,51 |
| 3 | H | $(C_2H_5)CHCH_3$ | H | L | 60 % | 147 - 49°C | ($CDCl_3$): 0,98 (t, 3H) 1,12 (d, 3H), 1,51 (m, 2H), 2,26 (m, 1H), 4,18 (d, 1H), 4,45 (m, 1H), 4,75 (m, 1H), 8,60 (br.s, 3H) | Ber.: C 34,49 H 6,06 N 5,61<br>Gef.: C 35,67 H 5,91 N 5,35 |

| Bsp. | $R^1$ | $R^2$ | $R^3$ | Stereo-chemie | Ausb. | Schmp. | $^1H$-NMR δ [ppm] | Analyse |
|---|---|---|---|---|---|---|---|---|
| 4 | H | $(CH_3)_3C-$ | H | L | 65 % | 136 - 38°C | (DMSO-$d_6$): 1,08 (s, 9H), 3,97 (s, 1H), 4,92 (m, 2H) 8,75 (br.s, 3H) | Ber.: C 38,49 H 6,06 N 5,61<br>Gef.: C 38,61 H 5,71 N 5,26 |
| 5 | H | $C_6H_5-CH_2-$ | H | L | 50 % | 130 - 33°C | (DMSO-$d_6$): 3,14 (dd, 1H) 3,29 (dd, 1H), 4,42 (m, 1H), 4,82 (m, 2H), 7,22 (m, 5H), 9,00 (br.s, 3H) | Ber.: C 46,57 H 4,62 N 4,94<br>Gef.: C 47,73 H 4,67 N 5,17 |
| 6 | $CH_3$ | $(CH_3)_2CH-$ | H | D,L | 84 % | 150 - 52°C | ($CDCl_3$): 1,09 (d, 3H) 1,11 (d, 3H), 1,76 (s, 3H), 2,31 (m, 1H), 4,62 (m, 2H), 9,05 (br.s, 3H) | Ber.: C 38,49 H 6,05 N 5,61<br>Gef.: C 38,84 H 5,77 N 5,28 |
| 7 | H | $Z-NH-(CH_2)_4-$<br>Z=Benzyloxycarbonyl | H | L | 68 % | 130 - 33°C | (DMSO-$d_6$): 1,20-1,80 (m, 6H), 2,96 (m, 2H), 4,14 (m, 1H), 4,85 (m, 2H), 5,05 (s, 2H), 7,38 (s, 5H), 9,20 (br.s, 3H) | Ber.: C 48,19 H 5,56 N 7,02<br>Gef.: C 48,18 H 5,62 N 6,64 |

| Bsp. | $R^1$ | $R^2$ | $R^3$ | Stereo-chemie | Ausb. | Schmp. | $^1H$-NMR $\delta$ [ppm] | Analyse |
|---|---|---|---|---|---|---|---|---|
| 8 | $-(CH_2)_5-$ | | H | - | 82 % | 181 - 83°C | ($CDCl_3$): 1,46 (m, 1H), 1,70 (m, 3H), 1,92 (m, 2H), 2,12 (m, 4H), 4,60 (q, 2H), 9,15 (br.s, 3H) | Ber.: C 41,31 H 5,78 N 5,35<br>Gef.: C 41,54 H 5,85 N 5,05 |
| 9 | H | $-(CH_2)_3-$ | | L | 30 % | Öl | ($CDCl_3$): 1,80-2,60 (m, 4H), 3,50 (m, 2H), 4,45 (m, 1H), 4,50-4,00 (m, 2H) | |

## Patentansprüche

1. α-Aminosäure-2,2,2-trifluorethylester der allgemeinen Formel I

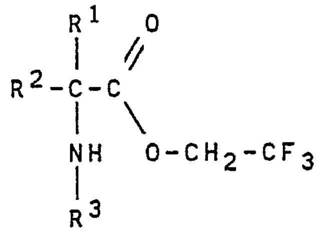

I

worin $R^1$ Wasserstoff oder $C_{1-4}$-Alkyl,

$R^2$ Wasserstoff, Alkyl, substituiertes Alkyl, Aryl, substituiertes Aryl, Heteroaryl, Arylalkyl, Heteroarylalkyl, Cycloalkyl- oder Cycloalkylmethyl ist,

oder

$R^1$ und $R^2$ zusammen mit dem sie verbindenden C-Atom einen 3 bis 7-gliedrigen gesättigten Ring bilden,

$R^3$ Wasserstoff oder $C_{1-4}$-Alkyl ist, mit der Maßgabe, daß für den Fall von $R^1$ = $CH_3$ $R^2$ und $R^3$ nicht Wasserstoff sind

oder

$R^2$ und $R^3$ zusammen mit dem sie verbindenden N- und C-Atom einen 4 bis 7-gliedrigen gesättigten Ring bilden

und

für den Fall $R^1 \neq R^2$ Racemate oder Enantiomere entstehen sowie die Säureadditonssalze davon.

2. α-Aminosäure-2,2,2-trifluorethylester nach Anspruch 1, dadurch gekennzeichnet daß

$R^1$ Wasserstoff oder $C_{1-3}$-Alkyl,

$R^2$ Wasserstoff, $C_{1-12}$-Alkyl, durch 1-3 Amino-und/oder Hydroxyl-und/oder Halogengruppen substituiertes $C_{1-12}$-Alkyl, wobei die Aminogruppen gegebenenfalls durch Acyl- oder Urethangruppen und die Hydroxylgruppen gegebenenfalls durch $C_{1-4}$-Alkyl- oder Benzylgruppen geschützt sind, Mono-, Di- oder Trihalogen-, Mono-, oder Di-trifluormethyl-, Mono-, Di- oder Trialkyl-Phenylgruppen, wobei Halogen Fluor, Chlor oder Brom und Alkyl $C_{1-4}$-Alkyl bedeutet, $C_{5-6}$-Ring-Systeme mit 1 bis 2 Heteroatomen bestehend aus O, N oder S, Benzyl, Cycloalkyl oder Cycloalkylmethyl mit 3-7 C-Atomen im Ring ist.

$R^3$ Wasserstoff oder $C_{1-3}$-Alkyl ist

oder

$R^2$ und $R^3$ zusammen mit dem sie verbindenden N-und C-Atom einen 4 bis 6-gliedrigen gesättigten Ring bilden.

3. α-Aminosäure-2,2,2,-trifluorethylester nach Anspruch 1 und 2, dadurch gekennzeichnet daß die Acylschutzgruppen aus Acetyl- oder Benzoyl und die Urethanschutz-Gruppen aus Methoxycarbonyl oder Benzyloxycarbonyl bestehen.

4. Verfahren zur Herstellung von α-Aminosäure-2,2,2-trifluorethylestern der allgemeinen Formel I, dadurch gekennzeichnet daß man ein N-Carbonsäureanhydrid der allgemeinen Formel II,

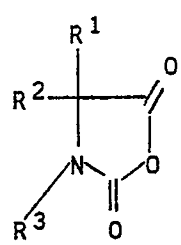

II

in der $R^1$, $R^2$, $R^3$ die unter Anspruch 1 bis 3 angegebene Bedeutung haben, wobei $R^2$ und $R^3$ auch Wasserstoff sein können, wenn $R^1$ = $CH_3$ ist, mit Lithium-, Natrium- oder Kalium-2,2,2-trifluorethanolat umsetzt.

5. Verwendung von Verbindungen gemäß Anspruch 1 bis 4 als Zwischenprodukte zur Herstellung von Agrochemikalien und von pharmazeutischen Wirkstoffen.

Europäisches Patentamt

# EUROPÄISCHER RECHERCHENBERICHT

Nummer der Anmeldung

**EP 91 10 3877**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,Y | EP-A-0 082 088 (B. ROQUES et al.)<br>* Beispiele 13,16,19 * | 1,2,5,4 | C 07 C 227/18<br>C 07 C 229/08<br>C 07 D 263/44 |
| Y | US-A-3 792 081 (T. HIGUCHI et al.) | 4 | |
| Y | US-A-4 622 413 (J.A. KROGH) | 4 | |
| D,A | CHEMICAL ABSTRACTS, Band 101, 1984, Seite 817, Zusammenfassung Nr. 192418r, Columbus, Ohio, US; V.F. POZDNEV: "Activation of carboxylic acids with pyrocarbonates. Acylation of primary hydroxyls using di(tert-butyl) pyrocarbonate-pyridine system as a condensing reagent", & BIOORG. KHIM. 1984, 10(7), 912-20<br>* Zusammenfassung * & TELESYSTEMSES QUESTEL ONLINE BILD VON CHEMICAL ABSTRACTS, Verbindungsnummer 92515-52-1 | 1 | |
| A | GB-A-1 211 625 (KYOWA HAKKO KOGYO) | 4 | |

RECHERCHIERTE SACHGEBIETE (Int. Cl.5)

C 07 C 227/00
C 07 C 229/00
C 07 D 623/00

Der vorliegende Recherchenbericht wurde für alle Patentansprüche erstellt

| Recherchenort | Abschlußdatum der Recherche | Prüfer |
|---|---|---|
| Den Haag | 03 Juli 91 | WELLS A.G. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument